# EUROPÄISCHE PATENTSCHRIFT

(11) **EP 1 765 306 B1**
(45) Veröffentlichungstag und Bekanntmachung des Hinweises auf die Patenterteilung: **14.11.2007**
(21) Anmeldenummer: 05759237.0
(22) Anmeldetag: 15.06.2005
(51) Int. Cl.: A61K 9/52

(54) **KAPSEL ZUM FREISETZEN VON IN IHR BEFINDLICHEN WIRKSTOFFEN AN DEFINIERTEN ORTEN IN EINEM KÖRPER**
CAPSULE FOR RELEASING AGENTS CONTAINED THEREIN AT DEFINED POINTS IN A BODY
CAPSULE POUR LA LIBERATION DE PRINCIPES ACTIFS CONTENUS DANS LADITE CAPSULE DANS DES ENDROITS DEFINIS D'UN CORPS

(30) Priorität: 13.07.2004 DE 102004034355
(43) Veröffentlichungstag der Anmeldung: 28.03.2007
(73) Patentinhaber: Fachhochschule Jena, 07745 Jena (DE)
(72) Erfinder: ANDRÄ, Wilfried, 07749 Jena (DE); BELLEMANN, Matthias Erich, 07743 Jena (DE); DANAN, Henri, F-67000 Strasbourg (FR); DUTZ, Silvio, 98744 Meura (DE); LIEBISCH, Stefan, 07673 Hartmannsdorf (DE); SCHMIEG, Rainer, 99444 Blankenhain (DE)
(74) Vertreter: Donath, Dirk
(86) Internationale Anmeldenummer: PCT/DE2005/001086
(87) Internationale Veröffentlichungsnummer: WO 2006/005287

(56) Entgegenhaltungen:
- WO-A-20/04078250
- DE-C1- 19 745 890
- US-A- 4 239 040
- US-A- 5 170 801

## Beschreibung

Die Erfindung betrifft eine Kapsel zum Freisetzen von in ihr befindlichen Wirkstoffen an definierten Orten in einem Körper gemäß der Gattung der Patentansprüche und soll insbesondere bei der Untersuchung des Verdauungstraktes verwendet werden.

Bekanntlich wird ein sehr großer Anteil aller Medikamente in Form von Tabletten oder Kapseln eingenommen, deren Wirkstoffe im Verdauungstrakt resorbiert werden. Mit Ausnahme des Magens, bei dem die Medikamenten-Applikation gut beherrscht wird, ist der genaue Ort der Resorption bisher noch nicht einstellbar. Es muss der Nachteil in Kauf genommen werden, dass die Passagegeschwindigkeit durch den Darm und der pH-Wert im Darm von Mensch zu Mensch und sogar beim selben Menschen abhängig von seinem Zustand starke Schwankungen aufweisen. Daher besteht selbst bei speziell präparierten Medikamenten-Kapseln, z. B. Zeit-gesteuerten, Enzym-gesteuerten, pH-Wert-gesteuerten oder Druck-gesteuerten Kapseln, die Gefahr, dass der Wirkstoff das Zielgebiet passiert, ohne in ausreichender Menge resorbiert zu werden. Werden Überdosierungen angewendet, besteht wiederum die Gefahr unverträglicher Nebenwirkungen. In der Vergangenheit sind eine Reihe von Verfahren, Anordnungen und Kapseln bekannt geworden, bei denen es sämtlich darum ging, den jeweiligen Ort einer Medikamenten-Kapsel im Darm zu bestimmen und - bei Erreichen des Zielgebietes - den Wirkstoff ferngesteuert aus der Kapsel freizusetzen, siehe hierzu Andrä, W. et al., A novel method for real-time magnetic marker monitöring in the gastrointestinal tract, Physics in Medicine and Biologie 45: 3081-3093 (2000); Hemmati, A., The Site of Iron Absorption in the Gastrointestinal Tract, German Med. Mth., Vol. XIII: 569 - 573 (1968); DE 29 28 477 A1; Gröning, R., Computer-controlled drug release from small-sized dosage forms, Journal of Controlled Release 48: 185-193 (1997); US 5170801 A; DE 197 45 890 A1; US 4239040 A; US 5279607 A. Die meisten der in den genannten Veröffentlichungen beschriebenen Kapseln besitzen mindestens einen der folgenden Nachteile:

Sie enthalten eine harte Hülle. Dadurch besteht die Gefahr, dass solche Kapseln an Stenosen im Darm stecken bleiben und möglicherweise operativ entfernt werden müssen. Diese Gefahr besteht, wie Rösch, T et al., in Derzeitige klinische Indikationen der Kapsel-Endoskopie, Zeitschrift für Gastroenterologie 40: 971-978 (2002) ausführen, sogar dann, wenn durch vorausgegangene Röntgen-Untersuchungen keine Stenosen festgestellt worden sind. Ferner sind in den genannten Kapseln harte Teile enthalten, wie z. B. Metallfedern, Batterien und elektronische Bauelemente oder Schaltkreise, die bei Kontakt mit der Darmwand toxisch wirken können.

Die eben genannten beiden Nachteile sind mit einer Darmtherapie-Kapsel gemäß DE 197 45 890 A1 und in gleicher Weise mit einer bereits vorgeschlagenen Kapsel mit rotierender Kugel bei Verwendung geeigneter Substanzen umgehbar. Dann besteht aber ein Nachteil, der im Mechanismus der Freisetzung begründet ist. Die Freisetzung wird dadurch bewirkt, dass ein Teilvolumen der Kapsel (im Folgenden als "Heiz-Element" bezeichnet) in einem magnetischen Wechselfeld durch magnetische Verluste oder durch Reibungsverluste soweit aufgeheizt wird, dass eine organische Substanz schmilzt bzw. eine Öffnung der Kapsel auf andere Weise ausgelöst wird. In der unmittelbaren Nachbarschaft des Heizelementes befindet sich dabei der Darminhalt oder die Darmwand bzw. der flüssige Wirkstoff. Die Wärmeleitfähigkeit dieser Umgebung ist so groß, dass bei zunehmender Erwärmung des Heizelementes immer mehr Wärme in die Umgebung abfließt und daher nicht zur Temperaturerhöhung des Heizelementes beiträgt.

Die maximal erreichbare Temperaturerhöhung ist dadurch bestimmt, dass die gesamte durch das Wechselfeld zugeführte Leistung in die Umgebung abfließt. Aus der Theorie der Wärmeleitung ergibt sich, dass die maximal erreichbare Temperaturerhöhung proportional der zugeführten Leistung und etwa umgekehrt proportional der Wärmeleitfähigkeit der Umgebung ist. Die Wärmeleitfähigkeit der Umgebung für die genannten Kapseln beträgt 0,2 W/(m·K) oder mehr. Der thermische Widerstand zwischen dem Heizelement und der Umgebung liegt in der Größenordnung von 1 bis 10 K/W. Die zugeführte Leistung des magnetischen Wechselfeldes muss genügend hoch gewählt werden, um trotz des Wärmeabflusses die gewünschte Maximal-Temperatur zu erreichen. Die zugeführte Leistung des magnetischen Wechselfeldes darf aber nicht beliebig hoch sein, da sonst durch Wirbelstrom-Verluste im Körpergewebe eine unzulässig hohe Erwärmung des Patienten eintreten kann [Brezovich, I.A., Low frequency hyperthermia: capacitive and ferromagnetic thermoseed methods, Medical Physics Monographs 16: 82-111 (1988)].

Der Erfindung liegt daher die Aufgabe zu Grunde, die oben genannten Nachteile durch eine neuartige Kapselgestaltung zu vermeiden. Es ist also eine Kapsel zu schaffen, die nicht Gefahr läuft, an Stenosen hängen zu bleiben, die aber andererseits energetisch günstig und thermisch verträglich ist.

Erfindungsgemäß wird diese Aufgabe durch die kennzeichnenden Merkmale des ersten Patentanspruchs gelöst und durch die Merkmale der Unteransprüche vorteilhaft ausgestaltet. Der thermische Widerstand des/der das Heizelement/die Heizelemente umgebenden Kapselteils/Kapselteile sollte wenigstens um eine Größenordnung höher sein als der thermische Widerstand der übrigen Kapselteile bzw. üblicher Medikamentenkapseln. Die Erfindung macht es möglich, dass einerseits alle Teile der Kapsel aus Substanzen bestehen, die bei Kontakt mit einem wässrigen Medium zerfallen oder sich auflösen, und dass andererseits der als Heizelement bezeichnete Kapselteil mit einer Hülle umgeben ist, die einen wesentlich höheren thermischen Widerstand als 10 K/W besitzt. Es wird die zum Erreichen der Freisetzungstemperatur erforderliche Leistung durch Einfügung einer thermischen Isolationshülle reduziert. Die unter dem Einfluss von magnetischen Wechselfeldern in mindestens einem Teil der Kapsel erzeugte Erwärmung führt dort zur ferngesteuerten Verdampfung einer leicht verdampfbaren Flüssigkeit, die den Wirkstoff (oder mehrere Wirkstoffe) aus dem Kapselinnenraum herausdrückt oder die aus Teilen bestehende Kapselwand bersten lässt. Hierzu ergibt sich eine vorteilhafte Lösung, bei welcher der das Heizelement zumindest teilweise umgebende Kapselteil durch eine hinsichtlich ihrer Lage und/oder ihrer Dehnung variable Wand gegenüber dem Wirkstoff verschlossen ist.

In einer bevorzugten Ausführungsform ist mindestens ein Heizelement, das ein magnetisches Pulver, bspw. Fe₃O₄ (Magnetit), enthält, zumindest teilweise mit einer thermisch isolierenden Hülle umgeben. Die Hülle kann doppelwandig sein, wobei die Wände aus wasserlöslichem Material, z. B. Hartgelatine oder Zucker, bestehen und durch eine Gasschicht, z. B. Luft, getrennt sind. Da die gesamte Kapsel nur eine begrenzte Größe besitzen darf, um beim Schluckvorgang keine Schwierigkeiten zu bereiten, ist die Dicke dieser Hülle ebenfalls begrenzt. Daher muss beim Vergleich der Wärmeleitfähigkeit ohne und mit Hülle diese Dicke berücksichtigt werden. Im optimalen Fall ergibt sich für die Hülle ein thermischer Widerstand von ca. 500 K/W im Verhältnis zu einer üblichen gleichgroßen Medizinkapsel, deren thermischer Widerstand etwa 10 K/W beträgt. Anstelle der doppelwandigen Hülle kann auch eine poröse Hülle aus wasserlöslichem Material mit eingeschlossenen Gas-Poren verwendet werden. Die Wärmeleitfähigkeit solcher poröser Stoffe und die Wärmeleitfähigkeit von Luft unterscheiden sich nur geringfügig.

Die Erfindung wird nachstehend an Hand der schematischen Zeichnung von vier Ausführungsbeispielen näher erläutert. Es zeigen:
- Fig. 1: einen Längsschnitt durch eine erfindungsgemäße Kapsel mit aufblähbarem Beutel,
- Fig. 2: einen Längsschnitt durch eine erfindungsgemäße Kapsel mit Stempel,
- Fig. 3: einen Längsschnitt durch eine erfindungsgemäße Kapsel mit verschiebbarer Trennwand und
- Fig. 4: einen Längsschnitt durch eine erfindungsgemäße Kapsel mit zwei Heizelementen und zwei lageveränderbaren Trennwänden.

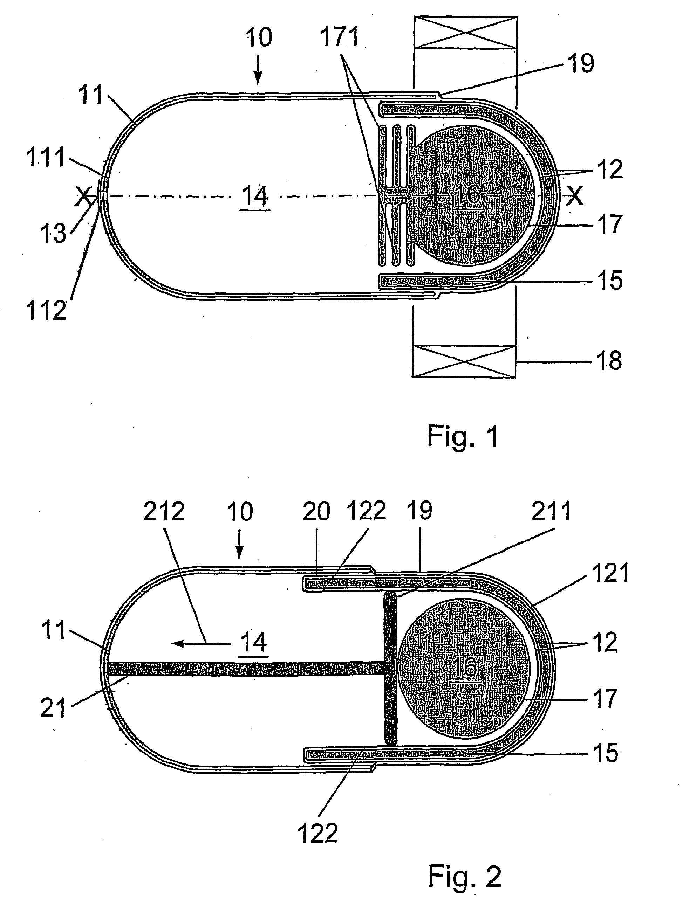

Fig. 1 zeigt eine Kapsel 10 aus zwei hermetisch dicht miteinander verbundenen, ineinander geschachtelten, bspw. aus Hartgelatine bestehenden Teilen 11, 12 mit einer gemeinsamen geometrischen Achse X-X. Der größere Teil 11 schiebt sich mit seinem offenen Ende über den kleineren Teil 12 und weist in einem zweidimensional gekrümmten Bereich 111 eine kleine Öffnung 112 auf, die mit einer Membran 13 gegen einen spontanen Austritt eines in der Kapsel 10 enthaltenen Wirkstoffs 14 verschlossen ist. Der Wirkstoff enthält üblicherweise kein Wasser. Die Öffnung 112 kann aber auch so klein oder selbst ventilartig so gestaltet sein, dass ein spontaner Austritt des Wirkstoffs 14 unter normalen Druckbedingungen nicht möglich ist. Der Kapselteil 12 ist doppelwandig und so ausgebildet, dass sich zwischen den Wänden Luft oder ein anderes geeignetes Isolationsmittel zur Wärmedämmung 15 befindet. Im Teil 12 ist ein mit einem Heizelement 16 gefüllter, dünnwandiger, aus Latex oder Polyethylen hergestellter Beutel 17 angeordnet, der durch eine Faltenstruktur 171 eine Volumenvergrößerung des Heizelements um mindestens 1 cm³ oder auf das Doppelte zulässt. Das Heizelement 16 besteht im vorliegenden Fall aus einer Mischung von ca. 40 Vol.% Fe₃O₄, dessen Verluste bei einem Ummagnetisierungszyklus etwa 1 J/kg betragen, und ca. 60 Vol-% Äthylalkohol, dessen Siedepunkt bei 78°C liegt und der bei dieser Temperatur verdampft und durch seine Ausdehnung in der Kapsel 10 einen Druck erzeugt, der die Öffnung 112 öffnet, so dass Wirkstoff 14 nach außen tritt. Statt des Äthylalkohols kann auch eine andere leicht verdampfende und biokompatible Flüssigkeit benutzt werden. Für die Temperaturerhöhung sorgt ein in bekannter Weise durch eine elektrische Spule 18. erzeugtes magnetisches Wechselfeld im Zusammenwirken mit den Fe₃O₄-Bestandteilen im Beutel 17.
Gegen eine Auflösung der Kapsel 10 in einer wasser- und/oder enzymhaltigen Umgebung, wie sie bspw. im Verdauungstrakt vorhanden ist, schützt eine die gesamte Kapsel 10 umhüllende dünne Schicht (Folie) 19 aus Polyethylen, Schellack oder einer anderen geeigneten Substanz. Die Öffnung 112 kann auch an einer anderen Stelle des Kapselteils 11 angeordnet sein. An Stelle des doppelwandigen Teils 12 ist auch ein Gasporen enthaltendes Bauteil aus wasserlöslichem Material verwendbar. Schließlich ist die Kapsel 10 weder an die in Fig. 1 dargestellte Form noch an die dort beschriebene Zweiteiligkeit gebunden. Der Ort der Spule 18 ist schematisch dargestellt; er befindet sich bei der Anwendung außerhalb des Körpers.

In Fig. 2 ist wieder eine Kapsel 10 dargestellt, deren Teile 11 und 12 durch eine die gesamte Kapsel 10 umgebende Folie 19 zusammen gehalten werden, die sich nicht zwischen den ineinander geschachtelten Teilen, also nicht in einem Bereich 20 befinden soll. An dieser Stelle kann zwischen den beiden Kapselteilen 11, 12 ein bioverträgliches Gleitmittel, bspw. Paraffin, eingefügt sein. In einem wärmedämmenden Teil 12 befindet sich wieder ein Beutel 17 mit einem Heizelement 16, das sich in unmittelbarer Nähe zum gekrümmten Bereich 121 des Teils 12 befindet und sich bei Ausdehnung des Heizelements 16 gegen den gekrümmten Bereich 121 abstützt. Außerdem weist der Teil 12 Führungsflächen 122 für Stütz- und Dichtelemente 211 eines Stempels 21 auf, der sich in einer durch einen Pfeil 212 gekennzeichneten axialen Richtung bewegen kann. Bei Erwärmung des Heizelements 16 in der zu Fig. 1 beschriebenen Weise die im Heizelement befindliche Flüssigkeit und drückt den Stempel 21 mit dem Teil 11 in der durch den Pfeil 212 gekennzeichneten Richtung. Die Folie 19 reißt im Bereich 20 auf, und der Wirkstoff 14 gelangt nach außen, bspw. in den Darmtrakt. Das eindringende Wasser o. dgl. löst die Teile 11 und 12 von innen heraus auf. Der Stempel 21 sollte ebenso wie die Stützelemente 211 aus einem wasserlöslichen Material bestehen.

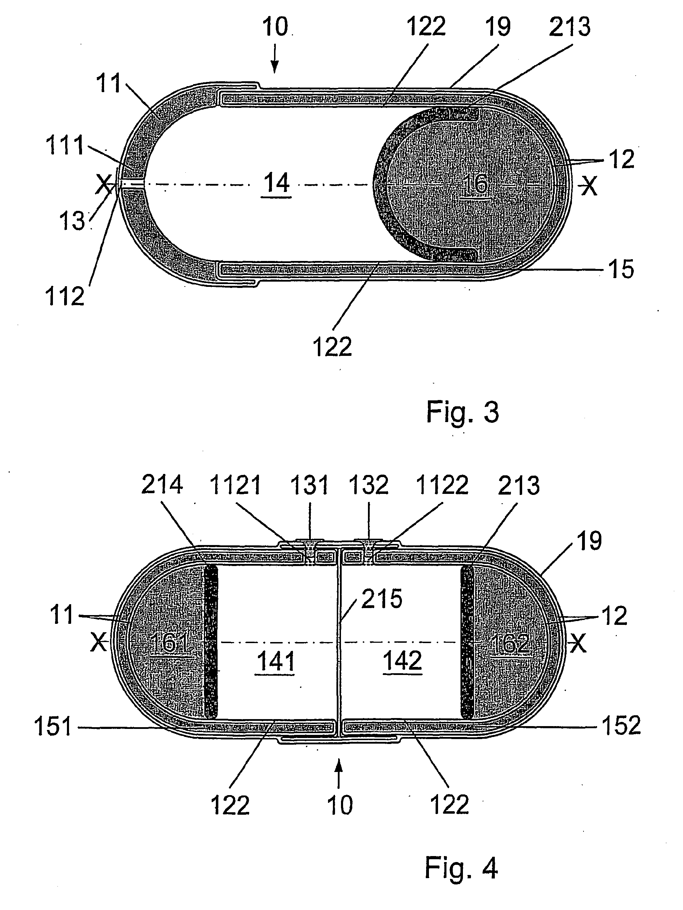

In Fig. 3 ist wieder eine zweiteilige Kapsel 10 dargestellt, deren mit einem Isolationsmittel 15 versehener Teil 12 in den mit einer Öffnung 112 versehenen Teil 11 axial geschoben und in diesem durch eine Folie 19 fixiert ist. Im Teil 12 befindet sich (vorzugsweise ohne den Beutel 17 der Fig. 2) ein Element 16, das unter der Wirkung eines von außen angelegten magnetischen Wechselfeldes als Heizelement wirkt und das durch eine verschiebbare Trennwand 213 vom Wirkstoff 14 getrennt ist, der den größeren Teil des Kapselinneren einnimmt. Bei der Erwärmung des Elements 16 dehnt sich dieses bspw. um das Doppelte aus und drückt auf den Wirkstoff 14 derart, dass eine Membran 13, welche die Öffnung 112 im Kapselteil 11 verschließt, zum Bersten gebracht wird. Durch die Öffnung 112 können der Wirkstoff 14 ausströmen und das Lösungsmittel für alle Kapselteile einschließlich der Trennwand 213 einströmen. Im übrigen gilt das zu den Figuren 1 und 2 Gesagte zumindest sinngemäß.

Die in dem Kapselteil 12 verschiebbare Trennwand 213 ist nicht an die in Fig. 3 dargestellte Konfiguration gebunden.

In Fig. 4 ist wieder eine zweiteilige Kapsel 10 dargestellt, deren aneinander stoßende beide Teile 11 und 12 mit Isolationsmitteln 151, 152 versehen sind, also einen höheren thermischen Widerstand aufweisen als die übrigen Kapselteile. In jedem der Teile 11 und 12 befindet sich ein Element 161 und 162, das unter der Wirkung eines von außen angelegten magnetischen Wechselfeldes als Heizelement wirkt und durch eine verschiebbare und/oder dehnbare Trennwand 213 und 214 von einem Wirkstoff 141 und 142 getrennt ist. Bei Erwärmung der betreffenden Elemente dehnen sich diese bspw. um das Doppelte aus und drücken ggf. nacheinander die Wirkstoffe durch Öffnungen 1121, 1122, die bspw. je mit einem Stopfen (Ventil) 131, 132 verschlossen sein können, nach außen. Die Heizelemente 161 und 162 können aus unterschiedlichen Materialien oder aus unterschiedlichen Mischungen aus leicht verdampfenden Flüssigkeiten und magnetischen Oxidpulvern bestehen, so dass die Verdampfung bei unterschiedlichen Temperaturen oder bei unterschiedlichen Leistungen des magnetischen Wechselfeldes erfolgt. Ferner können die Wirkstoffe 141 und 142 in den Kapselteilen 11, 12 unterschiedlich sein. Diese Wirkstoffe 141, 142 können auch so beschaffen sein, dass die gewünschte Wirkung erst nach ihrer Vermischung eintritt. Innerhalb der Kapsel 10 kann durch eine feste Trennwand 215 eine ungewollte Vermischung der beiden Wirkstoffe 141 und 142 verhindert werden.

Die vorstehend beschriebene Erfindung weist gegenüber dem Stand der Technik erhebliche Vorteile auf. Wegen des sehr viel größeren thermischen Widerstandes der Isolation des Teils 12 kann bei gleicher angestrebter Maximaltemperatur, bspw. 78°C, die der Kapsel 10 zugeführte Leistung des magnetischen Wechselfeldes wesentlich kleiner sein als im Fall der Kapseln ohne Isolationshülle. Obwohl wegen der Isolation das Volumen des Heizelementes kleiner wird, beträgt die erforderliche zugeführte Leistung für dieselbe Maximal-Temperatur im Idealfall weniger als 1 % der Leistung, die für die Kapsel ohne Wärmedämmung zugeführt werden müsste. Einen weiteren Vorteil bietet der dehnbare Beutel 17 bzw. eine biegsame und/oder verschiebbare Wand, die das Heizelement 16 gegen den Wirkstoff 14 abschließen. Sie vermeiden Kolben u. ä. aus hartem Material. Bei Erwärmung des magnetischen Pulvers über die Siedetemperatur der im Heizelement enthaltenen Flüssigkeit verdampft diese, und der Wirkstoff wird innerhalb kurzer Zeit ausgestoßen. Damit sind durch die Erfindung der Zeitpunkt und der Ort der Wirkstoff-Applikation besser definiert als mit den bekannten Verfahren und Vorrichtungen. Bei geeigneter Dosierung der zugeführten Wechselfeldleistung kann sogar der Wirkstoff in mehreren Portionen nacheinander appliziert werden. Der Aufbau der Kapsel 10 bewirkt, dass nach dem Ausstoßen des Wirkstoffs 14 die bspw. wasserhaltige Darmflüssigkeit in die Kapsel 10 eintritt und die Kapselteile 11, 12 bzw. den Stempel 21 aus Hartgelatine von innen her auflöst. Die anderen Kapselbestandteile (magnetisches Pulver, Polyethylen-Folie) sind leicht verformbar, so dass die Kapselreste an Stenosen nicht stecken bleiben können.

### Bezugszeichenliste

- 10: Kapsel
- 11, 12: (Kapsel-) Teile
- 13: Membran
- 14, 141, 142: Wirkstoffe
- 15, 151, 152: Luft, Isolationsmittel
- 16, 161, 162: Heizelemente
- 17: Beutel
- 18: Spule
- 19: Schicht, Folie
- 20: Bereich
- 21: Stempel
- 111, 121: gekrümmte Bereiche
- 112, 1121, 1122: Öffnungen
- 122: Führungsflächen
- 131, 132: Ventile, Stopfen
- 171: Faltenstruktur
- 211 1: Dicht- und Stützelemente
- 212: Pfeil
- 213,214: biegsame und/oder verschiebbare Trennwände
- 215: feste Trennwand
- X-X: Achse

## Patentansprüche

1. Kapsel zum Freisetzen von in ihr befindlichen Wirkstoffen an definierten Orten in einem Körper durch Erwärmung mindestens eines Heizelementes unter der Wirkung von mindestens einem magnetischen Wechselfeld, wobei sich die Kapsel bei Kontakt mit einer lösenden Flüssigkeit auflöst, **dadurch gekennzeichnet, dass** das Heizelement zumindest teilweise von einem Kapselteil umgeben ist, dessen Material einen höheren thermischen Widerstand aufweist als die übrigen Kapselteile.

2. Kapsel gemäß Anspruch 1, **dadurch gekennzeichnet, dass** das Heizelement ein Gemisch aus einem magnetischen Pulver und einer leicht verdampfenden Flüssigkeit ist.

3. Kapsel gemäß Anspruch 2, **dadurch gekennzeichnet, dass** das Heizelement ein Gemisch aus ca. 40 Vol-% Magnetit und 60 Vol-% Äthylalkohol ist.

4. Kapsel gemäß mindestens einem der Ansprüche 1 bis 3, **dadurch gekennzeichnet, dass** sich das Gemisch in einem Beutel befindet, der beim Verdampfen der Flüssigkeit sein Volumen mindestens verdoppeln kann.

5. Kapsel gemäß Anspruch 1, **dadurch gekennzeichnet, dass** der übrige Kapselteil mindestens eine Öffnung aufweist, durch die das erwärmte Heizelement den Wirkstoff nach außen presst und lösende Flüssigkeit einströmt.

6. Kapsel gemäß Anspruch 5, **dadurch gekennzeichnet, dass** die lösende Flüssigkeit Wasser ist.

7. Kapsel gemäß Anspruch 1, **dadurch gekennzeichnet, dass** alle ihre Teile von einer Schutzfolie gegen die lösende Flüssigkeit umgeben sind.

8. Kapsel gemäß Anspruch 1, **dadurch gekennzeichnet, dass** die Kapselteile teilweise ineinander geschachtelt sind.

9. Kapsel gemäß Anspruch 8, **dadurch gekennzeichnet, dass** die ineinander geschachtelten Kapselteile gemeinsame Berührungsflächen aufweisen, die frei von der Schutzfolie sind.

10. Kapsel gemäß Anspruch 9, **dadurch gekennzeichnet, dass** die gemeinsamen Berührungsflächen mit einem Gleitmittel versehen sind.

11. Kapsel gemäß mindestens einem der Ansprüche 1 bis 3, **dadurch gekennzeichnet, dass** das Heizelement über einen Stempel auf den übrigen Kapselteil wirkt.

12. Kapsel gemäß Anspruch 11, **dadurch gekennzeichnet, dass** der das Heizelement zumindest teilweise umgebende Kapselteil Führungsflächen für den Stempel aufweist.

13. Kapsel gemäß Anspruch 11, **dadurch gekennzeichnet, dass** beim Erwärmen des Heizmittels beide Kapselteile voneinander abgehoben werden.

14. Kapsel gemäß Anspruch 1, **dadurch gekennzeichnet, dass** zwischen dem Heizelement und dem Wirkstoff eine verschiebbare und/oder dehnbare Wand vorgesehen ist.

15. Kapsel gemäß Anspruch 1, **dadurch gekennzeichnet, dass** das Material mit dem höheren thermischen Widerstand Luft oder ein anderes Gas ist, das sich zwischen zwei Wänden aus Hartgelatine befindet.

16. Kapsel gemäß Anspruch 1, **dadurch gekennzeichnet, dass** das Material mit dem höheren thermischen Widerstand eine wasserlösliche poröse Substanz ist.

17. Kapsel gemäß Anspruch 1, **dadurch gekennzeichnet, dass** zwei Heizelemente diametral liegend angeordnet sind, deren Kapselteile mit höheren thermischen Widerständen aneinander stoßen.

18. Kapsel gemäß Anspruch 1 und 17, **dadurch gekennzeichnet, dass** die Heizelemente aus unterschiedlichen Materialien oder Materialmischungen bestehen.

19. Kapsel gemäß den Ansprüchen 1 und 17, **dadurch gekennzeichnet, dass** sie unterschiedliche Wirkstoffe enthält, die miteinander vermischt werden können.

## Claims

1. Capsule for releasing agents that are contained therein at defined positions in a body by heating at least one heating element under the effect of at least one alternate magnetic field, with said capsule dissolving when entering in contact with a dissolving liquid, wherein the heating element is at least partially surrounded by a capsule part the material of which has a greater thermal resistance than the other parts of the capsule.

2. Capsule as set forth in claim 1, wherein the heating element is a mixture of a magnetic powder and an easily evaporating liquid.

3. Capsule as set forth in claim 2, wherein the heating element is a mixture of ca. 40 Vol-% magnetite and 60 Vol-% ethyl alcohol.

4. Capsule as set forth in one of the claims 1 through 3, wherein the mixture is contained in a bag that can expand its volume to at least double the size when the liquid is evaporating.

5. Capsule as set forth in claim 1, wherein the other part of the capsule is provided with at least one opening through which the heated heating element presses the agent to the outside and the solving liquid enters the capsule.

6. Capsule as set forth in claim 5, wherein the solving liquid is water.

7. Capsule as set forth in claim 1, wherein all its parts are surrounded by a protective foil against the solving liquid.

8. Capsule as set forth in claim 1, wherein the capsule parts are partly nested one into the other.

9. Capsule as set forth in claim 8, wherein the capsule parts nested in each other have common contact surfaces that are not covered by the protective foil.

10. Capsule as set forth in claim 9, wherein the common contact surfaces are provided with a lubricant.

11. Capsule as set forth in at least one of the claims 1 through 3, wherein the heating element acts onto the other capsule part via a stamp.

12. Capsule as set forth in claim 11, wherein the capsule part that surrounds the heating element at least partially is provided with guide surfaces for the stamp.

13. Capsule as set forth in claim 11, wherein the two capsule parts are lifted one from the other when the heating element is heated.

14. Capsule as set forth in claim 1, wherein a movable and/or expandable wall is provided between the heating element and the agent.

15. Capsule as set forth in claim 1, wherein the material with the greater thermal resistance is air or another gas that is located between the two walls made of hard gelatin.

16. Capsule as set forth in claim 1, wherein the material with the greater thermal resistance is a water-soluble porous substance.

17. Capsule as set forth in claim 1, wherein two heating elements are contained in a diametral arrangement and their capsule parts with greater thermal resistances adjoin each other.

18. Capsule as set forth in claims 1 and 17, wherein the heating elements consist of different materials or mixtures.

19. Capsule as set forth in claims 1 and 17, wherein it contains different agents that can be mixed with each other.

## Revendications

1. Une capsule destinée à libérer dans un corps, à des endroits bien définis, les principes actifs se trouvant à l'intérieur de celle-ci, grâce au chauffage d'au moins un des éléments de chauffe sous l'influence d'au moins un champs magnétique alternant, la capsule se dissolvant après contact avec un liquide stimulant sa dissolution, est **caractérisée en ce que** l'élément de chauffe est entouré au moins en partie par une partie de capsule dont le matériau se distingue par une résistance thermique plus élevée que les autres parties de la capsule.

2. La capsule suivant la revendication 1 est **caractérisée en ce que** l'élément de chauffe est constitué d'un mélange composé d'une poudre magnétique et d'un liquide volatil.

3. La capsule suivant la revendication 2 est **caractérisée en ce que** l'élément de chauffe est constitué d'un mélange composé de 40 Vol- % de magnétite et de 60 Vol-% d'alcool éthylique.

4. La capsule suivant au moins une des revendications 1 à 3 est **caractérisée en ce que** le mélange se trouve dans une pochette qui, pendant l'évaporation du liquide, est en mesure d'au moins doubler son volume par extension.

5. La capsule suivant la revendication 1 est **caractérisée en ce que** l'autre partie de la capsule présente au moins un orifice à travers lequel l'élément de chauffe chauffé repousse le principe actif vers l'extérieur, le liquide dissolvant entrant dans la capsule.

6. La capsule suivant la revendication 5 est **caractérisée en ce que** le liquide dissolvant est de l'eau.

7. La capsule suivant la revendication 1 est **caractérisée en ce que** tous ses constituants sont enveloppés par une feuille les protégeant contre le liquide dissolvant.

8. La capsule suivant la revendication 1 est **caractérisée en ce que** le constituants de la capsule sont en partie emboîtés les uns dans les autres.

9. La capsule suivant la revendication 8 est **caractérisée en ce que** les constituants enchevêtrés de la capsule présentent des surfaces de contact communes qui ne sont pas protégées par de la feuille.

10. La capsule suivant la revendication 9 est **caractérisée en ce que** les surfaces de contact communes sont enduites d'un agent de glisse.

11. La capsule suivant les revendications 1 à 3 est **caractérisée en ce que** l'élément de chauffe agit par un poinçon sur l'autre partie de la capsule.

12. La capsule suivant la revendication 11 est **caractérisée en ce que** la partie de la capsule qui entoure au moins en partie l'élément de chauffe présente des surfaces de guidage pour le poinçon.

13. La capsule suivant la revendication 11 est **caractérisée en ce que**, pendant le chauffage du vecteur de chauffe, les deux parties de la capsule se séparent l'une de l'autre.

14. La capsule suivant la revendication 1 est **caractérisée en ce qu'**il est prévu de disposer entre l'élément de chauffe et le principe actif une cloison mobile et/ou élastique extensible

15. La capsule suivant la revendication 1 est **caractérisée en ce que** la matière présentant la résistance thermique supérieure est de l'air ou un autre gaz, situé entre deux parois de gélatine dure.

16. La capsule suivant la revendication 1 est **caractérisée en ce que** la matière présentant la résistance thermique supérieure est une substance poreuse soluble dans l'eau.

17. La capsule suivant la revendication 1 est **caractérisée en ce que** deux éléments de chauffe se trouvant en position diamétrale l'un par rapport à l'autre et dont les parties de la capsule présentent des résistances thermiques supérieures se jouxtent.

18. La capsule suivant la revendication 1 et 17 est **caractérisée en ce que** les éléments de chauffe sont constitués de matériaux ou de mélanges de matériaux différents.

19. La capsule suivant la revendication 1 et 17 est **caractérisée en ce qu'**elle contient dans son intérieur des principes actifs différents qui peuvent se mélanger l'un avec l'autre.
